(19) Europäisches Patentamt
European Patent Office
Office européen des brevets

(11) EP 1 506 235 B1

(12) EUROPEAN PATENT SPECIFICATION

(45) Date of publication and mention of the grant of the patent:
**30.05.2007 Bulletin 2007/22**

(21) Application number: **03725333.3**

(22) Date of filing: **17.04.2003**

(51) Int Cl.:
***C07K 16/00*** *(2006.01)* ***C12N 5/16*** *(2006.01)*
***C12N 15/06*** *(2006.01)*

(86) International application number:
**PCT/GB2003/001684**

(87) International publication number:
**WO 2003/089471 (30.10.2003 Gazette 2003/44)**

# (54) METHOD FOR PRODUCING MONOCLONAL ANTIBODIES

METHODE ZUR PRODUKTION MONOKLONALER ANTIKÖRPER

PROCEDE DE PRODUCTION D'ANTICORPS MONOCLONAUX

(84) Designated Contracting States:
**AT BE BG CH CY CZ DE DK EE ES FI FR GR HU IE IT LI LU MC NL PT RO SE SI SK TR**

(30) Priority: **17.04.2002 GB 0208817**

(43) Date of publication of application:
**16.02.2005 Bulletin 2005/07**

(73) Proprietor: **EUROPEAN MOLECULAR BIOLOGY LABORATORY**
**69012 Heidelberg (DE)**

(72) Inventors:
- **SAWYER, Alan, Michael**
  **European Molecular Biology**
  **D-69012 Heidelberg (DE)**
- **DE MASI, Federico**
  **European Molecular Biology**
  **D-69012 Heidelberg (DE)**

(74) Representative: **Goodfellow, Hugh Robin**
**Carpmaels & Ransford**
**43-45 Bloomsbury Square**
**London WC1A 2RA (GB)**

(56) References cited:
**WO-A-01/14425** **WO-A-96/33735**
**WO-A-03/019192**

- **TEMPLIN M F ET AL: "Protein microarray technology" TRENDS IN BIOTECHNOLOGY, ELSEVIER PUBLICATIONS, CAMBRIDGE, GB, vol. 20, no. 4, 1 April 2002 (2002-04-01), pages 160-166, XP004344214 ISSN: 0167-7799**
- **LUEKING A ET AL: "PROTEIN MICROARRAYS FOR GENE EXPRESSION AND ANTIBODY SCREENING" ANALYTICAL BIOCHEMISTRY, ACADEMIC PRESS, SAN DIEGO, CA, US, vol. 270, no. 1, May 1999 (1999-05), pages 103-111, XP000949937 ISSN: 0003-2697**
- **HAAB B B ET AL: "PROTEIN MICROARRAYS FOR HIGHLY PARALLEL DETECTION AND QUANTITATION OF SPECIFIC PROTENS AND ANTIBODIES IN COMPLEX SOLUTIONS" GENOME BIOLOGY (ONLINE), XX, GB, vol. 2, no. 2, 2001, page COMPLETE XP001147826 ISSN: 1465-6914**
- **ARENKOV P ET AL: "Protein microchips: use for immunoassay and enzymatic reactions" ANALYTICAL BIOCHEMISTRY, ACADEMIC PRESS, SAN DIEGO, CA, US, vol. 278, no. 2, 15 February 2000 (2000-02-15), pages 123-131, XP002234264 ISSN: 0003-2697**
- **EKINS R P: "LIGAND ASSAYS: FROM ELECTROPHORESIS TO MINIATURIZED MICROARRAYS" CLINICAL CHEMISTRY, AMERICAN ASSOCIATION FOR CLINICAL CHEMISTRY. WINSTON, US, vol. 44, no. 9, 1998, pages 2015-2030, XP002939389 ISSN: 0009-9147**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to methods for producing monoclonal antibodies. In particular, the invention relates to high throughput methods for producing monoclonal antibodies more rapidly than conventional methods.

The development of monoclonal antibody-producing cell lines by somatic fusions of B lymphocytes with myeloma cells was first described by Kohler and Milstein over 25 years ago (Kohler & Milstein, 1975). Since then, monoclonal antibodies have played a central role in the exponential growth of our understanding of human physiology, biochemistry and genetics. Monoclonal antibodies are versatile and sensitive tools for detecting and localising specific biological molecules. Monoclonal antibodies can be made against any cell molecule, enabling that molecule to be identified, localised and purified. In some cases, monoclonal antibodies also help identify the function of the molecules to which they bind.

The diagnostic and therapeutic potential of monoclonal antibodies was also quickly realised after the hybridoma technique allowed their development in the mid 1970s. Monoclonal antibodies have become key components in a vast array of clinical laboratory diagnostic tests. In addition, a large number of licensed drugs contain monoclonal antibodies and vast numbers of drugs in development are monoclonal antibodies. The clinical use of monoclonal antibodies has been improved by the development of chimeric and fully humanised monoclonal antibodies which minimise side-effects in patients.

However, despite the central role of monoclonal antibodies in these developments in medicine and molecular biology, the process for producing and screening monoclonal antibodies has changed little since it was first developed by Kohler & Milstein in the mid-1970s. The approach most often used to produce a monoclonal antibody against a specific antigen requires a series of immunisations of mice or rats with an antigen over the course of several weeks to enhance the activation and proliferation of mature B cells producing antigen-specific antibodies. Multiple mice are generally immunised and are tested periodically for the presence of relevant serum immunoglobulin titres prior to somatic fusion. Following fusion, the supernatants of hybridomas are screened using immunoassays to identify monoclonal antibodies with a high specificity for the antigen. The time frame required for developing a monoclonal antibody using this approach is generally 3 to 9 months.

WO 96/33735 discloses a method of producing a human antibody by immunizing a non-human transgenic animal with an antigen against which it is desired to produce the antibody. The non-human transgenic animal has been modified to produce B cells that secrete human antibodies instead of endogenous antibodies. Following immunization, B cells are obtained from the spleen of the animal and immortalized using Kohler & Milstein's technique to produce hybridomas. A sandwich ELISA in which the monoclonal antibody in the hybridoma supernatant is bound to antigen and to an anti-human constant region may be used to confirm that the antibody is human. Templin *et al.,* (Trends in Biotechnology, 2002, 20:160) is concerned with the theoretical advantages and limitations of microarray technology systems and, in particular, the changing use of protein microarrays in diagnostic methods and genome and proteome research.

The development of RIMMS (repetitive, multiple site immunisation strategy) has enabled somatic fusions to take place just 8-14 days after the initiation of immunisation (Kilpatrick *et al,* 1997). The supernatants of the hybridomas produced can then be screened using standard immunoassays, allowing a monoclonal antibody against a specific antigen to be isolated much more quickly

However, even using RIMMS, the production and screening of monoclonal antibodies against large numbers of different antigens requires considerable time and resources. As more and more novel proteins are discovered, there is a need for faster and more efficient methods for producing and screening monoclonal antibodies against these proteins, in order to allow their further characterisation.

## Summary of the invention

Accordingly, the invention provides a high-throughput method for producing a plurality of monoclonal antibodies, each of which binds to a different candidate antigen said method comprising the steps of:

a) introducing a plurality of candidate antigens into an animal or animals;
b) recovering antibody-producing cells from said animal or animals and rendering these cells into a single cell suspension;
c) generating immortalized cell lines from said single cell suspension;
d) screening the supernatant of said immortalized cell lines against a protein chip on which the candidate antigens are displayed; and
e) selecting monoclonal antibodies, that bind to said candidate antigens.

The method of the invention has considerable advantages over the methods of producing monoclonal antibodies that are currently available. As already discussed, current methods for producing monoclonal antibodies against more than one antigen involve laborious immunisation and isolation protocols for each individual antigen. In contrast, in the method of the invention, the animal may be injected with multiple antigens resulting in the simultaneous production of monoclonal antibodies against multiple antigens and increasing the speed and efficiency of monoclonal antibody production. The use of a protein chip in the method of the invention accelerates the process of screening to detect monoclonal antibodies that bind to the antigen or antigens with which the animal has been injected. In addition, the protein chip is more sensitive than conventional screening assays, such as enzyme linked immunosorbent assays (ELISAs), resulting in an improved detection rate for slow secreting hybridoma cells which would be missed using conventional screening methods. Additionally, the use of a protein chip in the method of the invention enables each supernatant to be screened multiple times against an antigen and uses only a fraction of the amount of antigen required for a single screening in a conventional screening assay such as an ELISA. For example, each supernatant can be screened in duplicate, triplicate or quadruplicate against an antigen.

The animal in step a) of the method of the invention may be any non-human mammalian animal. Preferably, the animal is a mouse, rat, rabbit, hamster or guinea pig. Preferably, the animal is a mouse.

The candidate antigen in step a) is preferably a purified candidate antigen. Either a purified candidate antigen or a mixture of purified candidate antigens may be introduced into the animal. By "purified candidate antigen" is meant that the antigen is a homogenous preparation of antigen that is substantially free from any other components. By "a mixture of purified candidate antigens" is meant that more than one purified antigen is present in the composition used for immunisation, but that the preparation is free from contaminating components for which there is no intention to elicit the production of antibodies. For example, although using conventional procedures, an animal may be immunised with multiple antigens simply by immunisation with homogenised tissue, such immunisation does not represent immunisation with purified candidate antigens as this is defined herein, since the antigens would be contaminated with cellular debris.

Any number of purified candidate antigens may be introduced into the animal. Preferably, more than one purified candidate antigens are introduced into the animal. For example, 2, 3, 4, 5, 6, 7, 8, 9, 10, 15, 20, 30, 40, 50 or more than 50 purified candidate antigens may be introduced into the animal. The antigens may be introduced simultaneously, in the sense that they are all mixed together. Alternatively, the antigens may be introduced separately one after the other. The introduction of different antigens may be separated by a time period of days. Preferably, the period separating the introduction of different antigens is less than 48 hours, preferably less than 24 hours. Preferably, the method of introduction involves injection of the antigen(s) into the animal.

By the term "candidate antigen" is meant any substance capable of inducing an immune response in an animal when that candidate antigen is introduced into the animal. The term therefore includes proteinaceous substances and non-proteinaceous substances.

Proteinaceous substances which are antigens include proteins and derivatives thereof, such as glycoproteins, lipoproteins and nucleoproteins and peptides. Fragments of such proteinaceous substances are also included within the term "antigen". Preferably, such fragments consist of or comprise antigenic determinants. Non-proteinaceous substances which are antigens include polysaccharides, lipopolysaccharides and nucleic acids. In particular, the term "antigen" includes nucleic acid molecules that induce an immune response against the proteins they encode. Fragments of such non-proteinaceous substances are also included with the term "antigen". The term "antigen" further includes proteinaceous or non-proteinaceous substances linked to a carrier which are able to induce an immune response, such as lipids or haptens upon which antigenicity is conferred when they are linked to a carrier. The antigens of the invention may be naturally occurring substances or may be synthesised by methods known in the art.

The antigen is preferably a proteinaceous substance or a nucleic acid molecule. Where the purified antigen is a proteinaceous substance, it may be introduced alone or in the form of a fusion protein. In particular, the invention provides that the antigen may be in the form of a fusion protein expressed on the surface of a recombinant virion with the animal being injected with the recombinant virion. The production of such recombinant virions using a nucleic acid sequence encoding the proteinaceous antigen, is described in Lindley et al, 2000.

Any combination of purified antigens may be used. The animal may be injected with only proteinaceous antigens, only non-proteinaceous antigens, or a mixture of both. According to one embodiment of the invention, the purified antigens are all proteinaceous. The purified antigens may be fragments derived from the same protein or different proteins. The purified antigens may be recombinant virions derived from a cDNA library, each recombinant virion expressing a protein encoded by a cDNA from the library on its surface.

According to a further embodiment, the invention provides that multiple purified antigens are introduced into the animal in the form of nucleic acid molecules encoding proteins against which it is desired to produce monoclonal antibodies. The nucleic acid molecules may be in DNA molecules, cDNA molecules or RNA molecules. Preferably, in this aspect of the invention, a cDNA library may be introduced into the animal. It is therefore possible to' inject the animal with nucleic acid molecules encoding a protein of unknown identity and as described below, cell chips may be used to isolate an antibody against the protein which in turn allows the protein to be purified.

Where the purified antigen is a nucleic acid molecule, it preferably consists of or comprises a DNA, cDNA or RNA sequence encoding a protein against which an immune response is to be induced. The nucleic acid molecule may be a naked nucleic acid molecule or it may be in the form of a vector.

The vector may be a viral vector, preferably a retroviral, adenoviral, adeno-associated viral (AAV), herpes viral, alphavirus vector or any other suitable vector as will be apparent to the skilled reader. Alternatively, the nucleic acid molecule may be in the form of a non-viral vector, preferably a plasmid vector. Many such vectors are known and documented in the art (see, for example, Fernandez J.M. & Hoeffler J.P. in Gene expression systems. Using nature for the art of expression ed. Academic Press, San Diego, London, Boston, New York, Sydney, Tokyo, Toronto, 1998). Such vectors may additionally incorporate regulatory sequences such as enhancers, promoters, ribosome binding sites and termination signals in the 5' and 3' untranslated regions of genes, that are required to ensure that the coding sequence is properly transcribed and translated.

Alternatively, the nucleic acid molecule may be in the form polycationic condensed DNA linked or unlinked to killed adenovirus alone, for example see Curiel (1992) *Hum Gene Ther* 3:147-154, or ligand linked DNA, for example see Wu (1989) *J Biol Chem* 264: 16985-16987. The nucleic acid molecule may also be in the form of DNA coated latex beads. Alternatively, the nucleic acid molecule may be encapsulated in liposomes as described, for example, in WO95/13796, WO94/23697, WO91/14445 and EP-524,968. *SA* 91 (24):11581-11585.

Antigen may be introduced into the animal by any suitable means. Preferably, the method of introduction involves injection. The animals may be immunised with the purified antigen or antigens intrasplenically, intravenously, intraperitoneally, intradermally or subcutaneously or by any other suitable means. The animals may be immunised with the purified antigen or antigens via more than one of these routes. For example, some of the purified antigen or antigens may be injected intraperitoneally and the rest subcutaneously. The means of injection will depend on the antigen or antigens being injected. For example, in the case of injection with a nucleic acid molecule a hand-held gene transfer particle gun, as described in US 5,149,655 can be used to inject the nucleic acid molecule.

In the case of a proteinaceous antigen, the dose of each antigen should preferably be in the range of between 0.01 and 1000 micrograms.

Preferably, the method of the invention comprises the additional step of supplying the animal with a booster dose of some or all of the antigens which are introduced into the animal prior to the recovery of the antibody-producing cells. The animals may be given a booster 1-365 days after the first injection. Preferably, the animals are boosted 1 to 20 times.

Preferably, the immunisation protocols used in the methodology of the present invention are short where more than one antigen is used in order to prevent one antigen becoming immunodominant. Preferably, where the animal is immunised with more than one antigen, it is injected with a booster of each antigen or combined booster of more than one antigen 3 days after the first injection and a further booster 5 days after the initial injection with spleen tissue or lymph nodes being removed between day 6 and day 15. Where a longer immunisation protocol is desired, the animal may be injected, for example, with a booster of each antigen or combined booster of more than one antigen 21 days after the first injection, with the spleen tissue or lymph nodes being removed on day 26.

Immunisation of the animal may be carried out with or without pharmaceutical carriers. Suitable carriers are typically large, slowly metabolized macromolecules such as proteins, polysaccharides, polylactic acids, polyglycolic acids, polymeric amino acids, amino acid copolymers, lipid aggregates (such as oil droplets or liposomes), and inactive virus particles. Such carriers are well known to those of ordinary skill in the art. Additionally, these carriers may function as immunostimulating agents ("adjuvants"). Immunisation of the animal may be carried out with or without adjuvants in addition to the pharmaceutical carriers.

Preferred adjuvants to enhance effectiveness of the composition include, but are not limited to: (1) aluminium salts (alum), such as aluminium hydroxide, aluminium phosphate, aluminium sulfate, *etc;* (2) oil-in-water emulsion formulations (with or without other specific immunostimulating agents such as muramyl peptides (see below) or bacterial cell wall components), such as for example (a) MF59™ (WO90/14837; Chapter 10 in *Vaccine design: the subunit and adjuvant approach,* eds. Powell & Newman, Plenum Press 1995), containing 5% Squalene, 0.5% Tween 80, and 0.5% Span 85 (optionally containing MTP-PE) formulated into submicron particles using a microfluidizer, (b) SAF, containing 10% Squalene, 0.4% Tween 80, 5% pluronic-blocked polymer L121, and thr-MDP either microfluidized into a submicron emulsion or vortexed to generate a larger particle size emulsion, and (c) Ribi™ adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); (3) saponin adjuvants, such as QS21 or Stimulon™ (Cambridge Bioscience, Worcester, MA) may be used or particles generated therefrom such as ISCOMs (immunostimulating complexes), which ISCOMS may be devoid of additional detergent e.g. WO00/07621; (4) Complete Freund's Adjuvant (CFA) and Incomplete Freund's Adjuvant (IFA); (5) cytokines, such as interleukins (*e.g.* IL-1, IL-2, IL-4, IL-5, IL-6, IL-7, IL-12 (WO99/44636) *etc.*), interferons (*e.g.* gamma interferon), macrophage colony stimulating factor (M-CSF), tumor necrosis factor (TNF), *etc.;* (6) monophosphoryl lipid A (MPL) or 3-O-deacylated MPL (3dMPL) *e.g.* GB-2220221, EP-A-0689454, optionally in the substantial absence of alum when used with pneumococcal saccharides *e.g.* WO00/56358; (7) combinations of 3dMPL with, for example, QS21 and/or oil-in-water emulsions e.g. EP-A-0835318, EP-A-0735898, EP-A-0761231; (8) oligonucleotides comprising CpG motifs (Roman *et al., Nat. Med.,* 1997, 3, 849-854; Weiner *et al., PNAS USA,* 1997, 94, 10833-10837; Davis *et al., J. Immunol.,* 1998, 160, 870-876; Chu *et al., J Exp. Med.,* 1997, 186, 1623-1631; Lipford *et al., Eur. J. Immunol.,* 1997, 27, 2340-2344; Moldoveanu *et al., Vaccine,* 1988, 16, 1216-1224, Krieg *et al., Nature,* 1995, 374, 546-549; Klinman *et al., PNAS USA,* 1996, 93, 2879-2883; Ballas *et al., J. Immunol.,* 1996, 157, 1840-1845; Cowdery *et al., J Immunol.,* 1996, 156, 4570-4575; Halpern *et al., Cell. Immunol.,* 1996, 167, 72-78; Yamamoto *et al., Jpn. J. Cancer Res.,* 1988, 79, 866-873; Stacey *et al., J Immunol.,* 1996, 157, 2116-2122; Messina *et al., J. Immunol.,* 1991, 147, 1759-1764; Yi *et al., J Immunol.,* 1996, 157, 4918-4925; Yi *et al., J. Immunol.,* 1996, 157, 5394-5402; Yi *et al., J. Immunol.,* 1998, 160, 4755-4761; and Yi *et al., J. Immunol.,* 1998, 160, 5898-5906; International patent applications WO96/02555, WO98/16247, WO98/18810, WO98/40100, WO98/55495, WO98/37919 and WO98/52581) *i.e.* containing at least one CG dinucleotide, with 5-methylcytosine optionally being used in place of cytosine; (9) a polyoxyethylene ether or a polyoxyethylene ester *e.g.* WO99/52549; (10) a polyoxyethylene sorbitan ester surfactant in combination with an octoxynol (WO01/21207) or a polyoxyethylene alkyl ether or ester surfactant in combination with at least one additional non-ionic surfactant such as an octoxynol (WO01/21152); (11) a saponin and an immunostimulatory oligonucleotide (*e.g.* a CpG oligonucleotide) (WO00/62800); (12) an immunostimulant and a particle of metal salt *e.g.* WO00/23105; (13) a saponin and an oil-in-water emulsion *e.g.* WO99/11241; (14) a saponin (*e.g.* QS21) + 3dMPL + IL-12 (optionally + a sterol) *e.g.* WO98/57659; (15) other substances that act as immunostimulating agents to enhance the effectiveness of the composition. Additional examples of adjuvants that may be used include Montanide ISA 50, Hunter's TiterMax, and Gerbu Adjuvants.

Preferred antibody-producing cells for use in the invention include B cells, T cells and stem cells. These antibody-producing cells for use in the invention may be recovered by removal of any suitable cellular components of the immune system from the animal. Preferably, antibody-producing cells are recovered from the animal by removal of the spleen, lymph nodes or bone marrow or portions thereof. These may be rendered into a single cell suspension according to step b) of the method of the invention via any suitable means. Preferably, spleen tissue, lymph nodes or bone marrow removed from the animal are rendered into a single cell suspension by mechanical disruption or enzymatic digestion with proteases. Red cells may be removed from the cell suspension by hypotonic lysis.

Preferably, the immortalized cell line specified in step c) of the method of the invention is a hybridoma cell line produced by somatic fusion of the cells in the single cell suspension to myeloma cells. Cells in the single cell suspension are fused to myeloma cells with a fusogen. Examples of myeloma cells which may be used include SP2, NS1 and NS0. Preferably, the fusogen is PEG, a virus or a method of electrofusion (Zimmermann et al. 1990).

The hybridoma cells produced by the fusion of the single cell suspension with the myeloma cells should be cultured. Preferably, the hybridoma cells are initially cultured in a selective media, such as Azaserine hypoxanthine or Hypoxanthine aminopterin thymidine, and are then transferred to a non-selective media. Preferably, the hybridoma cells are cultured on selective media for 7 days and are then transferred to a non-selective media for 3 days. This ensures that the growth rate of the cells increases prior to the screening step. Preferably, the steps involved in hybridoma production are conducted robotically in order to speed up the process. The Examples set out one way of conducting the steps involved in hybridoma production robotically.

Alternatively, the immortalized cell line may be a cell line generated by infection of cells in the single cell suspension with an immortalizing virus. Preferably, the immortalizing virus is Epstein-Bar virus (see, for example, Epstein Barr Virus Protocols, Eds. Wilson and May, Humana Press; ISBN: 0896036901).

Step d) of the method of the invention comprises screening the supernatant of the immortalized cell line, preferably a hybridoma cell line, against a protein chip comprising a candidate antigen with which the animal was immunised. As used herein, the term "protein chip" is used to encompass any microarray made up of a supporting means to which a candidate antigen has been anchored.

Where more than one purified antigen has been introduced into the animal, each purified antigen may be displayed at a different position on the protein chip, preferably at a predetermined position. Each position on the protein chip may thus display a different antigen. Alternatively, the same antigen may be anchored to each position in a row or column of a protein chip with a different antigen being displayed in each row or column. In some cases, it may be desirable, even when the animal has been immunised with more than one purified antigen, to have a different chip for each antigen. A protein chip may have a large number, such as between 1 and 1000 purified antigens, anchored at predetermined positions on a chip.

Any type of protein chip known in the art may be used in the method of invention. For example, the protein chip may be a glass slide to which the purified antigen or antigens are anchored. Where only one antigen is being tested, such a slide may be prepared simply by coating glass microscope slides with aminosilane (Ansorge, Faulstich), adding an antigen-containing solution to the slide and drying. Slides coated with aminosilane may be obtained from Telechem and Pierce for coating with the purified antigen. Preferably, such a glass slide may be coated with (6-aminohexil) aminosilane.

Other types of protein chips which may be used in the method of the invention include a 3D gel pad (Arkenov *et al,* 2000) and microwell chips. As will be apparent to the skilled reader, types of protein chips that have not yet been conceived but which are devised in the future may well prove to be suitable for use in accordance with the present invention.

The term "protein chip" also includes microarrays of cells expressing defined cDNAs (Ziauddin *et al,* 2001) referred to herein as "cell chips". In this technique, mammalian cells are cultured on a glass slide printed in defined location with different cDNAs. Cells growing on the printed locations take up and express the cDNAs. Cell chips are particularly useful when the animal has been injected with a cDNA or a cDNA library or with a recombinant virion or virions produced from a cDNA library, as described above. In such cases, the proteins encoded by the cDNA sequences may not have been isolated. By injecting the animal with cDNAs encoding the proteins or recombinant virions expressing the cDNAs, it is possible to produce monoclonal antibodies against the proteins expressed by the cDNAs. If the same cDNAs are expressed using a cell chip, these antibodies will bind and the binding may be detected as described below. Providing that a nucleic acid sequence encoding the protein is available, the invention in this manner enables the detection and isolation of a monoclonal antibody against that protein which may be used to purify the protein itself.

For selection of antibodies, or selection of immortalised cell lines producing such antibodies, the supernatant from the immortalised cell line or cell lines is spotted onto the protein chip or protein chips at defined positions on the chip. Spotting of supernatants is preferably done robotically, for example with a Genemachines Ominigrid arrayer using Telechem pins. Preferably, the supernatants spotted onto the protein chip or protein chips contain glycerol to minimise drying and fixing of the antibodies on the slide. For example, 0 to 99.9% glycerol may be used. The chip is then washed to remove any unbound supernatant. At this stage, any monoclonal antibody produced by the immortalized cell line and hence in the supernatant may be bound to an antigen on the chip.

By using different elution conditions, the method allows the approximate quantification of the binding affinity of the monoclonal antibody for its binding partner. Elution agents that may be used include chaotropic agents such as guanidine hydrochloride or urea at concentrations between 10 $\mu$molar and 8 molar or ethylene glycol in an aqueous solution of 0.01% to 100% w/v. Elutions may also be carried out using aqueous or non-aqueous solutions of glycine at concentrations of between 0.01 molar and a saturated solution (preferably 200mM), at a pH of between pH9 and pH1, preferably pH3.2. High pH elutions may be carried out using aqueous or non aqueous solutions of triethylamine between 1 $\mu$molar and a saturated solution, preferably 100mM, at a pH of between pH8 and pH13, preferably pH 11.5.

Step e) of the method of the invention involves selection of a monoclonal antibody that binds to the antigen. Preferably, this step incorporates a detection step, such as by adding a marker which will bind to bound monoclonal antibody and indicate its presence. Preferably, the marker is labelled with a label such as an enzymatic or fluorescent label that enables its presence to be detected. For example, the marker may be labelled protein A or labelled protein G. Protein A or protein G may be labelled with a fluorescent label such as Cy3 or Cy5. Alternatively, protein A or protein G may be labelled with an enzymatic label such as biotin, the presence of which can be detected by the binding of labelled strepavidin or avidin.

Preferably, the marker is an antibody that will bind to the first antibody. Preferably this antibody is labelled with a label such as an enzymatic or fluorescent labels. Preferably this antibody is labeled with fluorescent labels as this enables equipment developed for scanning of DNA chips to be used for detection.

Preferably, the step of detecting a monoclonal antibody bound to the antigen further comprises isotyping the monoclonal antibodies. Preferably, this step of detecting and isotyping the monoclonal antibodies comprises adding isotype-specific anti-immunoglobulin antibodies to said protein chip, wherein each anti-immunoglobulin antibody having a different isotype specificity has a different label, and detecting the presence of said labels. This method enables the simultaneous detection of the monoclonal antibody and determination of its isotype.

It will be appreciated that the method may employ as many different isotype-specific anti-immunoglobulin antibodies, each with a different label, as there are antibody isotypes in the animal which has been immunised. For example, if a mammal, such as a mouse, has been injected with an antigen, the step of detecting and isotyping monoclonal antibodies bound to the antigen may comprise adding an anti-IgA antibody labelled with a first label, and/or an anti-IgD antibody labelled with a second label, and/or an anti-IgE antibody labelled with a third label, and/or an anti-IgG1 antibody labelled with a fourth label, and/or an anti-IgG2a antibody labelled with a fifth label, and/or an anti-IgG2b antibody labelled with a sixth label, and/or an anti-IgG3 antibody labelled with a seventh label, and/or an anti-IgG4 antibody labelled with a eighth label, and/or an anti-IgM antibody labelled with a ninth label. Alternatively, the step of detecting and isotyping monoclonal antibodies bound to the antigen may comprise adding isotype-specific anti-immunoglobulin antibodies that bind to a subset of the possible isotypes. Preferably, the isotype-specific anti-immunoglobulin antibodies comprise an anti-IgM antibody labelled with a first label and an anti-IgG antibody labeled with a second label. Preferably, the labels are fluorescent labels.

Detection of the label indicates the presence of a monoclonal antibody bound to an antigen and is preferably done robotically. Where the label is a fluorescent label, detection of the label and hence the presence of the monoclonal antibody may be done using equipment available for scanning protein chips. For example, scanning of the chips may be done with a GenePix 4000B scanner (Axon Instruments, Inc.) or with a Tecan LS200 or LS400 scanner. Scanning may be carried out with between 1 and 4 lasers and combinations of filters to enable visualisation of multiple fluorescent labels. Preferably, visualisation of multiple fluorescent labels is carried out simultaneously although it may be carried out sequentially.

Images may be obtained and analysed using appropriate software such as the GenePix Pro software (Axon Instruments, Inc.), Chipskipper software (Schwager, Ansorge) or Tecan LS200 or LS400 software.

In order to ensure that the detection of a monoclonal antibody is reliable, the screening method preferably employs various controls. For example, in the case of a protein chip coated with one antigen, not only will the supernatants from the immortalized cell lines produced by the method of the invention be spotted onto the protein chip but so will positive and negative controls.

Positive controls may be in the form of previously tested monoclonal antibodies or commercially available polyclonals. Alternatively, positive controls may consist of diluted or undiluted serum previously collected from the immunized mouse either a suitable period after the boost or at the moment the animal is sacrificed for the collection of the source of B-cells.

Negative controls may be in the form of mock supernatants at defined positions. Another level of control is determined by the fact that each supernatant is screened against several antigens. Signals obtained against only one antigen are considered to be potential positive monoclonal antibody containing supernatants.

Positive signals on the protein chip can be traced back to a particular immortalised cell line enabling the monoclonal antibody to be isolated according to step e) of the method of the invention. Further characterisation of the antibodies identified can then be carried out.

Methods for carrying out further characterisation of the antibody may include, for example, the further step of determining the specificity of the monoclonal antibodies identified. For example, a monoclonal antibody identified by the method of the invention may be used to scan a second protein chip having a large number of different proteins anchored to its surface to establish if the monoclonal antibody binds specifically to one antigen. The scanning methods described above in the initial identification of the protein may be used to scan for its specificity. The binding specificity and affinity of the monoclonal antibodies produced by the method of the invention may be further characterised by altering the concentrations of antigen on protein chips or altering the stringency of eluting conditions, as described above.

According to a further embodiment of the first aspect of the invention, there is provided a method for producing a bank of immortalised cell lines that produce a plurality of monoclonal antibodies of interest, said method comprising the steps of:

a) introducing a plurality of candidate antigens into an animal or animals;
b) recovering antibody-producing cells from said animal or animals and rendering these cells into a single cell suspension;
c) generating immortalized cell lines from said single cell suspension;
d) screening the supernatant of said immortalized cell lines against a protein chip on which the candidate antigens are displayed; and
e) selecting immortalised cell lines, that produce supernatants containing antibodies that bind to said candidate antigens.

Immortalised cell lines produced by such a method are of immense utility in the generation of antibodies with tailored specificities.

The candidate antigens are preferably purified candidate antigens, as described above. Suitable procedures for introducing the candidate antigens into the animal, recovering antibody-producing cells, generating immortalized cell lines and screening the supernatants of the immortalized cell lines are described above.

Prior art methods involve laborious and time-consuming procedures to generate and screen for a monoclonal antibody against a single antigen. In contrast, this method enables the generation and high-throughput screening of monoclonal antibodies against a plurality of antigens simultaneously. The use of a protein chip to conduct high-throughput screening of the antibodies is more efficient and more accurate than the use of conventional assays and requires less candidate antigen.

Preferably, step e) of this embodiment further comprises isotyping the monoclonal antibodies, as described above. This provides an additional advantage over the prior art methods which do not disclose simultaneous detection and isotyping of monoclonal antibodies.

The invention provides monoclonal antibodies. The invention may also be used to generate a bank of antibodies, for example, with specificities encompassing an entire organismic proteome. Such a bank of antibodies represents a further aspect of the invention.

The invention also provides immortalized cell lines; preferably hybridoma cell lines, which produce monoclonal antibodies. The invention also provides a bank of immortalized cell lines, preferably a bank of hybridoma cell lines. The invention may also be used to generate a bank of hybridoma cell lines, for example, that produce antibodies encompassing an entire organismic proteome.

According to a fourth aspect of the invention, there is provided a method for producing a plurality of monoclonal antibodies, each of which binds to a different purified candidate antigen, comprising introducing a plurality of purified candidate antigens into an animal.

Preferably, each candidate antigen is derived from a different source. By this is meant that each antigen is derived from a different protein, a different nucleic acid and so on. It is intended that methods of antibody production that involve injecting an animal with different fragments of the same protein are excluded from the scope of this aspect of the invention. For example, the purified candidate antigens may all be proteinaceous substances provided that they are not all fragments of the same protein.

This method has an advantage over methods disclosed in the prior art in that it enables the simultaneous production of more than one monoclonal antibody, each of which binds to a different purified candidate antigen.

The animal may be injected with the purified candidate antigens using any of the techniques described herein. For example, the method of this aspect of the invention may further comprise the steps of recovering antibody-producing cells such as B cells, T cells and stem cells from an immunised animal, such as by removing spleen tissue, lymph nodes or bone marrow, and rendering them into a single cell suspension. The method may further comprise generating immortalized cell lines, preferably hybridoma cell lines, from the cells the single cell suspension. Preferably, the method of this aspect of the invention comprises these steps and additionally comprises the steps of screening the supernatants of the immortalized cell lines, preferably hybridoma cell lines, against a protein chip or protein chips on which the candidate antigens are displayed; and selecting monoclonal antibodies that bind to the antigens and preferably isolating these and/or the immortalized cell lines that produce the monoclonal antibodies. Suitable procedures for generating the immortalized cell lines and subsequent screening of the supernatants are the same as those described in above in connection with the method of the first aspect of the invention. In particular, the step of detecting the monoclonal antibodies may involve simultaneous detection of the monoclonal antibodies and determination of this isotype, as described above. In addition, the method may comprise further characterisation of the monoclonal antibodies, as described above.

The invention also provides a monoclonal antibody produced by a method of this aspect of the invention. Again, this aspect of the invention may be used to generate a bank of antibodies, for example, encompassing antibodies with specificity for protein in an entire organismic proteome. Such a bank of antibodies represents a further aspect of the invention.

The invention also provides an immortalized cell line, preferably a hybridoma cell line, which produces a monoclonal antibody as described above. The invention may also be used to generate a bank of immortalized cell lines, preferably a bank of hybridoma cell lines, for example, that produce antibodies encompassing an entire organismic proteome.

According to a fifth aspect of the invention, anti-idiotype antibodies may be generated that bind to a monoclonal antibody according to the second aspect of the invention. Anti-idiotype antibodies are useful as they have a variable region that mimics the shape of the molecule to which the original antibody was raised. Anti-idiotype antibodies may therefore be useful in therapy as replacements for the molecules against which the original antibody was raised. An anti-idiotype antibody may be produced by employing the method of the first aspect of the invention or the fourth aspect of the invention using a monoclonal antibody according to the second aspect of the invention as the purified candidate antigen.

Accordingly, this aspect of the invention provides a method of producing an anti-idiotype antibody that binds to a monoclonal antibody according to the second aspect of the invention, the method comprising using a monoclonal antibody according to the second aspect of the invention as a purified candidate antigen in a method of the first aspect of the invention or the fourth aspect of the invention. The invention also includes anti-idiotype antibodies generated by such methods.

According to a sixth aspect of the invention, anti-anti-idiotype antibodies may be generated that bind to an anti-idiotype antibody produced according to the fifth aspect of the invention. Such anti-anti-idiotype antibodies may be produced by employing the method of the first aspect of the invention or the fourth aspect of the invention using an anti-idiotype antibody as described above as the purified candidate antigen. This aspect of the invention thus provides a method of producing an anti-anti-idiotype antibody that binds to an anti-idiotype antibody generated according to the fifth aspect of the invention, the method comprising using an anti-idiotype antibody as described above as a purified candidate antigen in a method of the first aspect of the invention or the fourth aspect of the invention.

Various aspects and embodiments of the present invention will now be described in more detail by way of example. It will be appreciated that modification of detail may be made without departing from the scope of the invention.

**Brief description of the Figures:**

Figure 1: Whole image of scanned chip, where green and red spots represent positive IgG and IgM producing supernatants respectively. Close ups are to show details of specific areas of chip where good spots are to be found.

Figure 2: Comparison between chip analysis and ELISA screen. First image is negative sample (Ia <0.5), while others are positive. Average Ic: Average total intensity of spot on 3 chips. Ia: Average contribution of spot to sum of intensities over three chips (%).

Figure 3: Comparison of contribution to total intensity (%) of culture supernatants screened by ELISA (■) or by chip analysis (□) for binding to B5 antigen (Figure 3A). The background values are shown in Figure 3B. A number of positive supernatants identified by chip analysis and/or ELISA are shown.

Figure 4: Comparison of contribution to total intensity (%) of culture supernatants screened by ELISA (■) or by chip analysis (□) for binding to B5 antigen (Figure 4A). The background values are shown in Figure 4B. A single positive supernatant identified by chip analysis and ELISA is shown.

Figure 5: Comparison of contribution to total intensity (%) of culture supernatants screened by ELISA (■) or by chip analysis (□) for binding to Ket94/95 antigen (Figure 5A). The background values are shown in Figure 5B. A number of positive supernatants identified by chip analysis and/or ELISA are shown.

Figure 6: Comparison of contribution to total intensity (%) of culture supernatants screened by ELISA (■) or by chip analysis (□) for binding to Ket94/95 antigen (Figure 6A). The background values are shown in Figure 6B. No positive supernatants were identified by either ELISA or chip analysis.

## Examples

### Example 1: Immunization with 10 protein antigens

### Immunization

An 8-week old female Balb/c mouse was injected intrasplenically with 10 μg of each of 10 protein antigens in 100μl phosphate buffered saline (PBS). On the third day following (day 3) the mouse was injected intraperitoneally with 1ug of each of the same 10 protein antigens in 100μl PBS. On day 5, the same mouse was injected intravenously with 0.1μg of each of the same 10 protein antigens. On Day 8 the mouse was killed by cervical dislocation and the spleen removed and collected into Dulbecco's Modified Eagle's Medium (DMEM: Life Technologies Inc.).

### Fusion

All steps are performed under sterile or aseptic conditions in a laminar flow hood. The spleen was rendered into a single-cell suspension by mechanical disruption between two frosted-end glass microscope slides. The suspension was filtered into a 50ml bar-coded conical-bottomed tube (BD Falcon) through a 70μm nylon cell strainer (BD Falcon) and transferred to the robotic system.

Separately, SP2 myeloma fusion partners (ATCC) were cultured for five days prior to fusion in HM20 (DMEM, 20% Defined foetal bovine serum (Hyclone Defined), 10mM L-Glutamine, 50μM Gentamicin) and on the day of the fusion were transferred to HM20/HCF/2xOPI (HM20 containing 10% Hybridoma Cloning Factor (Origen) and 2%OPI cloning supplement (Sigma)) for at least one hour at 37°C in a 5% $CO_2$ incubator.

The bar codes were read by a bar code reader and the 50ml Falcon tube loaded into the rotor by the RoMa arm on the genesis Freedom system (Tecan). The rotor was loaded into the centrifuge through the workdeck.

The tube was centrifuged at 100g for 10 mins at room temperature (RT) and the rotor extracted from the centrifuge. The tube was extracted from the rotor and the bar code was again read to distinguish from the balance tube. Cells were resuspended in 5ml Red Cell Lysis Buffer (Sigma) for 9 minutes at RT. HM20 was added to 50ml and the tube once again centrifuged for 10 min at RT with no brake. The supernatant solution was aspirated to waste and the cells resuspended in DMEM preheated to 37°C. Cells were washed twice more by steps of centrifugation and resuspension. 50μl of cell suspension were robotically pipetted to a 1.5ml microcentrifuge tube. Cells were counted using a haemocytometer counting chamber.

Simultaneously the SP2 cells were washed three times in a similar fashion and a similar aliquot (50μl) "handed off" to a 1.5 ml tube for haemocytometric counting. SP2 myelomas and spleen cells were mixed at a ratio of 1:5 (SP2:Spleen) and again centrifuged at 100g for 10 min with no brake.

The supernatant solution was entirely aspirated to waste and Polethyleneglycol 1500 in 50% HEPES (PEG: Roche Molecular Biochemicals) pre-heated to 37°C was robotically pipetted smoothly and progressively over 1 min with rotation at 450rpm on a Te-shake shaker (Tecan AG) to ensure even mixing. The cell/PEG mixture was incubated for 1 min at 37°C with gentle agitation. 1 ml of DMEM was similarly added over 1 min at 37°C with similar agitation. The mixture was incubated for 1 min at 37°C with gentle agitation. A further 1ml of DMEM was robotically added over 1 min at 37°C with gentle agitation and incubated similarly for a further minute. 7mls of HM20 were robotically added over 3 min at 37°C with gentle agitation. The Tube was then spun at 90g for 5 min with brake. The supernate was aspirated to waste and the pellet resuspended in 20ml of HM20/HCF/OPI/AH (HM20/HCF/OPI plus 10% Azaserine Hypoxanthine (Sigma).

The conical tube was again placed on the robot workdeck and the post-fusion cell slurry was aspirated by each of the 8 wide-bore pipette tips of the liquid handling arm of the robot. 200μl of the cell slurry was then pipetted into each well of a 96-well deep well plate (Greiner Masterblock).

The deep-well plate was then robotically transferred to a TeMo 96-well pipetting robot integrated onto the Genesis work-deck and used as a source plate to plate out into the 20 sterile 96-well tissue culture plates.

The post-fusion mixture was then robotically plated out into 20 96-well sterile plates (Nunc) sourced from a carousel attached and integrated to the robot at 100μl/well and robotically transferred to an integrated 37°C incubator with 10% $CO_2$ through the integrated airlock. Plates were stored in a carousel contained with the incubator.

**Cell Culture**

On the third day following the fusion cells were robotically transported from the incubator to the work deck and a further 100μl HM20/HCF/OPI/AH was robotically added. The plates were then robotically returned to the incubator.

On day 7 the plates were once again similarly transported from incubator to work deck and 200μl/well of culture supernates was aspirated to waste and replaced with 150μl fresh HM20/HCF.

On day 11 the plates were again robotically transported to the work deck and 30μl of supernate was collected from each well (Temo head: Tecan Inc.) and transferred to 384-well plates supplied to the workdeck by a carousel plate stacker (Tecan Inc).

**Microarray screening**

Aminosilane coated glass slides were homogeneously coated with purified antigen by dropping 40μl of $ddH_2O$ containing 1-5 μg of antigen and covering with a 22*60mm coverslip for 60min in a humid chamber at RT.

Coated slides were rinsed briefly in PBS and blocked for 60' in 5% milk in PBS, 0.1% Tween, then washed for 10' in PBS. The chips were then dried by centrifugation, 10" at 2000 rpm.

Culture supernatants were consolidated into 384 well plates using a Beckman Biomek FX robot.

Culture supernatants were printed singularly onto three identical antigen-coated slides at a density of 9600 spots per chip and a spot size of ~120μm using a GeneMachines OmniGrid microarray printer.

The microarray chips were incubated in a humid chamber for 60', at RT and then washed 5 x 5' in PBS-0.1% Tween (PBST)

40μl of Cy3 conjugated goat anti-mouse IgG-specific and Cy5 conjugated goat anti-mouse IgM-specific antibodies were diluted 1:1000 in PBST, mixed and applied uniformly to the chips and covered with 22x60 mm coverslips and incubated in a humid chamber, for 30' at RT. The chips were then washed 2 x 10' in PBST, 2 x 10' in PBS and 1 x 10' in $ddH_2O$. The chips were dried by centrifugation at 2000 rpm for 10".

**Hit-Picking**

Chips were scanned with a GenePix 4000B scanner (Axon Instruments), at a resolution of 10 um.pixel$^{-1}$. PMT voltages were 540V and 610V, for the Cy3 and Cy5 channels respectively. Both lasers were set at 100% intensity. Each scanned chip was assigned a fitted grid, and all spots were analysed by the GenePix Pro 3.0 (Axon Instruments).

All chips were analysed by the GenePix Pro 3.0 software and we collected, for each chip, the information relative to the corrected intensity ($I_c$) of each spot (Median of intensities - Background), for the Cy3 and Cy5 channels. From this data, we obtained, for each chip, the contribution of each spot to the total Corrected Intensity of each channel $\left(\left(I_c \Big/ \sum_1^{9600} I_c\right)\times 100\right)$. The three values of each channel were averaged for each spot, and this Average Corrected Intensity ($I_a$) was used for the final analysis of the dataset. We considered as "likely to be positive" samples the ones that have a value above 0.5% and as "sure positives", all the samples showing a value superior to 1.5%.

**Post Screening Processing**

Cells from positive wells were resuspended in the well and 20μl transferred to a 96-deep-well plate previously filled with 1.5mls of HM20/HCF and returned to the incubator for 48hrs at 37°C, 5%$CO_2$.

1.4ml of culture each supernate was transferred to another deep well plate and the remaining 100μl used to resuspend the cells which were then transferred to a freezing vial containing 90% Foetal bovine serum/ 10%DMSO (Sigma) and transferred to -80°C for 2hrs and from there to liquid nitrogen store. Culture supernate was then used for evaluation and further characterization of the generated monoclonal antibody.

**Results:**

The results of microarray screening are shown in Figure 1. This Figure demonstrates that a number of positive monoclonal antibodies were detected as binding to candidate antigens on the slide. The green spots are IgG monoclonal antibodies which bind to the candidate antigens while the red spots are IgM monoclonal antibodies which bind to the candidate antigens. Figure 1 thus demonstrates that the method of the invention can be used to simultaneously identify monoclonal antibodies that bind to the candidate antigens and the isotypes of those monoclonal antibodies.

When a comparative experiment was conducted using ELISA in the place of a protein chip, a number of monoclonal antibodies identified as binding to candidate antigens in the microarray screening were not identified as binding to candidate antigens using ELISA, as shown in Figure 2 (see comparison of microarray results with ELISA results). A supernatant which was found to be negative using microarray screening (Ia:0.234) was also found to be negative using ELISA. However, of four supernatants found to be positive using microarray screening (Ia: 5.18, 1.96, 3.64 and 2.02), only two were found to be positive using ELISA (see Figure 2). It is important to note how the supernatants that were negative in the ELISA experiment can be actual positive samples as detected by the more accurate and sensitive microarray approach.

### Example 2: Immunization with nine antigens

#### Immunization:

A mouse was injected with 25μg of nine antigens, including 25μg of a fusion of the antigens B5 and Ket94/95, each antigen being mixed with 10μg CpG DNA and adsorbed onto alum adjuvant (Imject Alum from Pierce). Half of each antigen was administered intraperitoneally and half subcutaneously.

The mouse was boosted 21 days later with 10μg of each antigen mixed with 10μg of CpG DNA and adsorbed onto alum adjuvant, half of which was administered intraperitoneally and half subcutaneously.

Five days after the boost, the spleen was removed.

#### Fusion and cell culture

The fusions and cell culture were performed as described in Example 1.

#### Microarray screening of antibodies against B5 and Ket94/95

An aminosilane glass slides was homogenously coated with purified B5 by dropping 40μl of ddH2O containing 5μg of purified B5 and covering with a 22*60mm coverslip for 60 min at room temperature. The same procedure was used to produce an aminosilane glass slide homogenously coated with purified Ket94/95.

The coated slides were rinsed, blocked, washed and dried, as described in Example 1, except that 3% BSA in PBS was used in place of 5% milk in PBS to block the slide.

Culture supernatants were consolidated into 384 well plates, as described in Example 1 and were printed in triplicate onto the slide coated with B5 and the slide coated with Ket94/95 at a density of around 16000 spots per chip and a spot size of ~150μm using a Microgrid II 610 microarray printer (ApogentDiscoveries).

The microarray chips were incubated and Cy3 conjugated goat anti-mouse IgG-specific and Cy5 conjugated goat anti-mouse IgM-specific antibodies were applied to the chips as described in Example 1.

#### Hit picking

The chips were scanned using the same procedure as in Example 1. An Average Corrected Intensity (Ia) was calculated for each set of triplicate culture supernatants.

#### Comparison of microarray screening and ELISA

A comparative experiment was conducted in which each culture supernatant was checked by ELISA. Each culture supernatant was added to a well containing 200ng of B5 or Ket94/95 antigen and the presence of a monoclonal antibody that bound to the antigen in the culture supernatant was detected using a conventional ELISA.

One week was required to screen 5376 culture supernatants using ELISA, producing 5376 results, one for each supernatant. In contrast, it took under 48 hours to screen the same 5376 culture supernatants in triplicate using a microarray chip, producing 16128 results, demonstrating the efficiency of the microarray screening compared to ELISA.

Furthermore, only 5μg of B5 antigen and 5μg of Ket94/95 was required to carry out the microarray screening, 5μg antigen per chip. In contrast, 96μg of antigen was required for each ELISA plate and five ELISA plates were required to screen each antigen. Screening by ELISA was thus significantly more costly and time-consuming that microarray screening.

The data obtained for each ELISA plate was normalised to provide a percentage contribution to total intensity for each culture supernatant. The averaged replicate intensities for the same culture supernatants obtained by microarray screening were also normalised to allow comparison with the ELISA data.

When culture supernatants were screened using ELISA for monoclonal antibodies binding B5, 53 positive supernatants were identified over five ELISA plates. Screening of the same culture supernatants using microarray screening identified 48 positive supernatants, 90.57% of the supernatants identified by ELISA. Microarray screening also identified 4 novel positives that were not identified by ELISA.

When culture supernatants were screened using ELISA for monoclonal antibodies binding Ket94/95, 15 positive supernatants were identified on a single ELISA plate. Screening of the same culture supernatants using microarray screening identified 13 positive supernatants, 88.66% of the positive supernatants identified by ELISA. Microarray screening also identified 8 novel positives that were not identified by ELISA.

It Is clear from these results that microarray screening is at least as effective as ELISA at identifying monoclonal antibodies that bind a specific antigen. Indeed the identification of positive supernatants not identified by ELISA suggests that microarray screening is more sensitive than ELISA. Microarray screening further had the significant advantage that it allowed simultaneous determination of the IG or IgM isotype of the monoclonal antibodies identified.

Figure 3A shows the normalised values of percentage contribution to total intensity for each culture supernatant in an ELISA plate (■) containing positive samples that bind B5 compared to the normalised values of percentage contribution for the same culture supernatants obtained by microarray screening of a B5-coated slide (□). Figure 3B shows the level of background noise in these experiments. It can be seen that positive supernatants showed a greater percentage contribution to total intensity using microarray screening compared to ELISA. As a result, there was a greater difference between background noise and a positive supernatant in microarray screening compared to ELISA, enabling positive supernatants to be identified more easily and more accurately.

Figure 4A compares the normalised values of percentage contribution to total intensity for each culture supernatant in an ELISA plate (■) containing a single positive sample that binds B5 compared to the normalised values of percentage contribution for the same culture supernatants obtained by microarray screening on a B5-coated slide (□). The level of background noise is shown in Figure 4B and it can be seen that positive sample was more readily detectable above the background noise using microarray screening compared to ELISA.

Figure 5A compares the normalised values of percentage contribution to total intensity for each culture supernatant in the ELISA plate found to contain positive supernatants that bind KET94/95 (■) compared to the normalised values of percentage contribution for the same culture supernatants obtained by microarray screening on a KET94/95-coated slide (□). The positive supernatants were more readily detectable above the background noise using microarray screening compared to ELISA, as shown in Figure 5B.

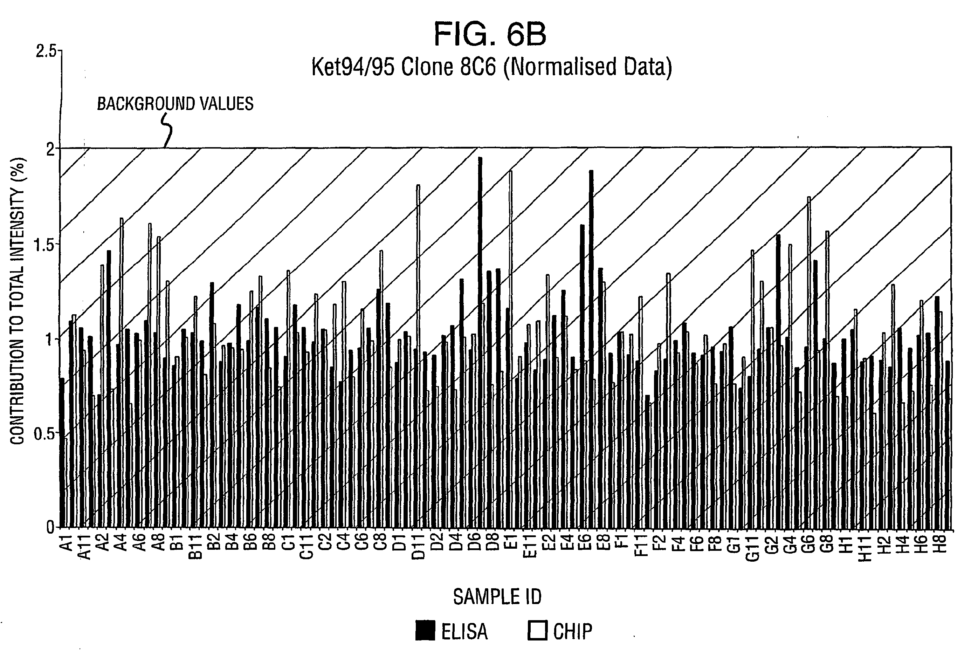

Figures 6A compares the data obtained from an ELISA plate (■) in which there were no positive supernatants to data obtained using microarray screening (□) of the same culture supernatants. As shown in Figure 6B, the readings in both cases were due to background noise.

These results demonstrate that the method of the invention can be used to simultaneously identify monoclonal antibodies against more than one antigen. The use of microarray screening in the method of the invention is quicker, cheaper and more accurate than the use of conventional antibody screening methods, such as ELISA.

**References**

Kilpatrick *et al* (1997) Hybridoma 16: 381-389

Kohler G, Milstein C (1975) Nature 7: 256:485-7

Lindley et al (2000) J Immunol Methods 234: 123-135

Ziaudin J, Sabatini D (2001) Nature 411: 107-110

Zimmermann U. (1990) J Immunol Methods Nov 6; 134(1):43-50

**Claims**

1. A high-throughput method of producing a plurality of monoclonal antibodies, each of which binds to a different candidate antigen, said method comprising the steps of:

   a) introducing a plurality of purified candidate antigens into an non-human animal or non-human animals;
   b) recovering antibody-producing cells from said animal or animals and rendering these cells into a single cell suspension;
   c) generating immortalized cell lines from said single cell suspension;
   d) screening the supernatants of said immortalized cell lines against a protein chip on which the candidate antigens are displayed; and
   e) selecting monoclonal antibodies that bind to said candidate antigens.

2. The method of claim 1 wherein said animal is a mouse, a rat, a guinea pig or a rabbit.

3. The method of claim 1 or claims 2 wherein between two and fifty different purified candidate antigens are introduced into the animal or animals.

4. The method of claim 3 wherein between 0.001 and 1000 micrograms of each antigen is introduced into the animal or animals.

5. The method of any one of claims 1 to 4 comprising the additional step of supplying the animal or animals with a booster dose of some or all of the antigens which were introduced into the animal or animals prior to the removal of antibody-producing cells.

6. The method of any one of claims 1 to 5 wherein the antibody-producing cells are B cells, T cell or stem cells.

7. The method of any one of claims 1 to 6 wherein the antibody-producing cells are recovered by removal of spleen tissue, lymph nodes or bone marrow of the animal or animals.

8. The method of any one of claims 1 to 7 wherein the immortalized cell line is a hybridoma cell line produced by somatic fusion of the cells in the single cell suspension to myeloma cells.

**9.** The method of any one of claims 1 to 8 wherein said protein chip is a plain-glass slide, a 3D gel pad chip, a microwell chip or a cell chip.

**10.** The method of any one of claims 1 to 9 wherein the step of detecting the monoclonal antibodies bound to the antigens further comprises isotyping the monoclonal antibodies.

**11.** The method of claim 10 wherein said step of detecting and isotyping the monoclonal antibodies comprises adding isotype specific anti-immunoglobulin antibodies to said protein chip, wherein each anti-immunoglobulin antibody having a different isotype specificity has a different label, and detecting the presence of said labels.

**12.** The method of any one of claims 1 to 11 further comprising assessing the specificity with which each isolated monoclonal antibody binds to an antigen using a protein chip comprising said antigen.

**13.** A method of identifying a plurality of monoclonal antibodies, each of which binds to a different candidate antigen, said method comprising the steps of:

a) screening the supernatant of immortalised cell lines against one or more protein chips on which the candidate antigens are displayed; and
b) selecting as said monoclonal antibodies, antibodies that bind to said candidate antigens,

said method being **characterised in that** said immortalised cell lines are generated from a single suspension of antibody-producing cells that produce antibodies against a plurality of antigens.

**14.** A method of producing a bank of immortalised cell lines that produce a plurality of monoclonal antibodies of interest, each of which binds to a different candidate antigen, said method comprising the steps of:

a) introducing a plurality of different antigens into an non-human animal or non-human animals;
b) recovering antibody-producing cells from said animal or animals and rendering these cells into a single cell suspension;
c) generating immortalized cell lines from said single cell suspension;
d) screening the supernatant of said immortalized cell lines against a protein chip on which the candidate antigens are displayed; and
e) selecting as an immortalised cell lines, that which produce supernatants containing antibodies that binds to said candidate antigens.

**Patentansprüche**

**1.** Hochdurchsatzverfahren zur Herstellung einer Vielzahl monoklonaler Antikörper, von denen jeder an einen verschiedenen Antigenkandidaten bindet, wobei das Verfahren die Schritte umfasst:

a) das Einführen einer Vielzahl gereinigter Antigenkandidaten in ein nicht humanes Tier oder nicht humane Tiere,
b) das Rückgewinnen Antikörper-produzierender Zellen aus dem Tier oder den Tieren und das Vereinen dieser Zellen in eine Einzelzellsuspension,
c) das Erzeugen immortalisierter Zelllinien aus der Einzelzellsuspension,
d) das Screenen des Überstands der immortalisierten Zelllinien gegen einen Protein-Chip, auf welchem die Antigenkandidaten dargestellt sind, und
e) das Auswählen monoklonaler Antikörper, welche an die Antigenkandidaten binden.

**2.** Verfahren nach Anspruch 1, wobei das Tier eine Maus, eine Ratte, ein Meerschweinchen oder ein Kaninchen ist.

**3.** Verfahren nach Anspruch 1 oder Anspruch 2, wobei zwischen zwei und fünfzig verschiedene gereinigte Antigenkandidaten in das Tier oder die Tiere eingeführt werden.

**4.** Verfahren nach Anspruch 3, wobei zwischen 0,001 und 1000 Mikrogramm jedes Antigens in das Tier oder die Tiere eingeführt werden.

**5.** Verfahren nach einem der Ansprüche 1 bis 4, umfassend den zusätzlichen Schritt des Versorgens des Tieres oder der Tiere mit einer Boosterdosis von einigen oder allen der Antigene, welche vor dem Entfernen Antigenproduzierender Zellen in das Tier oder die Tiere eingeführt wurden.

**6.** Verfahren nach einem der Ansprüche 1 bis 5, wobei die Antikörperprodzierenden Zellen B-Zellen, T-Zellen oder Stammzellen sind.

**7.** Verfahren nach einem der Ansprüche 1 bis 6, wobei die Antikörper-produzierenden Zellen durch das Entfernen von Milzgewebe, Lymphknoten oder Knochenmark des Tieres oder der Tiere rückgewonnen werden.

**8.** Verfahren nach einem der Ansprüche 1 bis 7, wobei die immortalisierte Zelllinie eine Hybridom-Zelllinie ist, welche durch somatische Verschmelzung der Zellen in der Einzelzellsuspension zu Myelomzellen hergestellt wird.

9. Verfahren nach einem der Ansprüche 1 bis 8, wobei der Protein-Chip ein einfacher Glas-Objektträger, ein 3D Gelpolster-Chip, ein Mikrovertiefungs-Chip oder ein Zell-Chip ist.

10. Verfahren nach einem der Ansprüche 1 bis 9, wobei der Schritt des Detektierens der an die Antigene gebundenen monoklonalen Antikörper weiter das Isotypisieren der monoklonalen Antikörper umfasst.

11. Verfahren nach Anspruch 10, wobei der Schritt des Detektierens und Isotypisierens der monoklonalen Antikörper das Hinzufügen Isotyp-spezifischer Antiimmunglobulin-Antikörper zu dem Protein-Chip, wobei jeder Antiimmunglobulin-Antikörper mit einer verschiedenen Isotyp-Spezifität eine verschiedene Markierung aufweist, und das Detektieren der Gegenwart der Markierungen, umfasst.

12. Verfahren nach einem der Ansprüche 1 bis 11, weiter umfassend das Bewerten der Spezifität, mit welcher jeder isolierte monoklonale Antikörper an ein Antigen bindet, unter Verwendung eines das Antigen umfassenden Protein-Chips.

13. Verfahren zum Identifizieren einer Vielzahl monoklonaler Antikörper, von denen jeder an einen verschiedenen Antigenkandidaten bindet, wobei das Verfahren die Schritte umfasst:

a) das Screenen des Überstands immortalisierter Zelllinien gegen einen oder mehrere Protein-Chip(s), auf welchem die Antigenkandidaten dargestellt sind, und
b) das Auswählen als die monoklonalen Antikörper von Antikörpern, welche an die Antigenkandidaten binden,

wobei das Verfahren **dadurch gekennzeichnet ist, dass** sie immortalisierten Zelllinien aus einer Einzelsuspension von Antikörper-produzierenden Zellen, welche Antikörper gegen eine Vielzahl von Antigenen herstellen, erzeugt werden.

14. Verfahren zur Herstellung einer Bank von immortalisierten Zelllinien, welche eine Vielzahl monoklonaler Antikörper von Interesse bilden, von denen jeder an einen verschiedenen Antigenkandidaten bindet, wobei das Verfahren die Schritte umfasst:

a) das Einführen einer Vielzahl verschiedener Antigenkandidaten in ein nicht humanes Tier oder nicht humane Tiere,
b) das Rückgewinnen Antikörper produzierender Zellen aus dem Tier oder den Tieren und das Vereinen dieser Zellen in eine Einzelzellsuspension,
c) das Erzeugen immortalisierter Zelllinien aus der Einzelzellsuspension,
d) das Screenen des Überstands der immortalisierten Zelllinien gegen einen Protein-Chip, auf welchem die Antigenkandidaten dargestellt sind, und
e) das Auswählen als immortalisierte Zelllinien, die Überstände bilden, welche an Antigenkandidaten bindende Antikörper enthalten.

**Revendications**

1. Procédé à haut débit d'obtention d'une pluralité d'anticorps monoclonaux, dont chacun se fixe à un antigène candidat différent, ledit procédé comprenant les étapes de :

a) introduire une pluralité d'antigènes candidats purifiés chez un animal non humain ou des animaux non humains ;
b) récupérer les cellules produisant des anticorps à partir dudit animal ou desdits animaux et amener ces cellules dans une suspension à cellule unique ;
c) générer des lignées de cellules immortalisées à partir de ladite suspension à cellule unique ;
d) cribler les surnageants desdites lignées de cellules immortalisées contre une puce de protéine sur laquelle des antigènes candidats sont affichés ; et
e) sélectionner les anticorps monoclonaux qui se fixent auxdits antigènes candidats.

2. Procédé selon la revendication 1, dans lequel ledit animal est une souris, un rat, un cochon d'Inde ou un lapin.

3. Procédé selon la revendication 1 ou la revendication 2, dans lequel on introduit chez l'animal ou les animaux entre deux et cinquante antigènes candidats purifiés différents.

4. Procédé selon la revendication 3, dans lequel on introduit chez l'animal ou les animaux entre 0,001 et 1 000 microgrammes de chaque antigène.

5. Procédé selon l'une quelconque des revendications 1 à 4, comprenant l'étape supplémentaire de fourniture à l'animal ou aux animaux d'une dose renforçatrice de certains ou de tous les antigènes qui sont introduits chez l'animal ou les animaux avant l'élimination des cellules produisant des anticorps.

6. Procédé selon l'une quelconque des revendications 1 à 5, dans lequel les cellules produisant des anticorps sont des cellules B, des cellules T ou des cellules souches.

7. Procédé selon l'une quelconque des revendications 1 à 6, dans lequel les cellules produisant des anticorps sont récupérées par élimination de tissus de rate, ganglions lymphatiques ou moelle d'os de l'animal ou des animaux.

8. Procédé selon l'une quelconque des revendications 1 à 7, dans lequel la lignée de cellules immortalisées est une lignée de cellules hybridomes produite par fusion somatique des cellules dans la suspension à cellule unique aux cellules de myélome.

9. Procédé selon l'une quelconque des revendications 1 à 8, dans lequel ladite puce de protéine est une plaquette de verre lisse, une puce à tampon de gel 3D, une puce à micropuits ou une puce de cellule.

10. Procédé selon l'une quelconque des revendications 1 à 9, dans lequel l'étape de détection des anticorps monoclonaux liés aux antigènes comprend, en outre, l'isotypage des anticorps monoclonaux.

11. Procédé selon la revendication 10, dans lequel ladite étape de détection et d'isotypage des anticorps monoclonaux comprend l'ajout d'anticorps anti-immunoglobuline spécifiques d'isotypes à ladite puce de protéine, dans lequel chaque anticorps anti-immunoglobuline ayant une spécificité d'isotype différent a une étiquette différente et la détection de la présence desdites étiquettes.

12. Procédé selon l'une quelconque des revendications 1 à 11, comprenant, en outre, la détermination de la spécificité avec laquelle chaque anticorps monoclonal isolé se fixe à un antigène en utilisant une puce de protéine comprenant ledit antigène.

13. Procédé d'identification d'une pluralité d'anticorps monoclonaux dont chacun se fixe à un antigène candidat différent, ledit procédé comprenant les étapes de :

   a) criblage du surnageant des lignées de cellules immortalisées contre une ou plusieurs puces de protéines sur laquelle/lesquelles les antigènes candidats sont affichés ; et
   b) sélection comme lesdits anticorps monoclonaux, d'anticorps qui se fixent auxdits antigènes candidats,

   ledit procédé étant **caractérisé en ce que** lesdites lignées de cellules immortalisées sont générées à partir d'une seule suspension de cellules produisant des anticorps qui produisent des anticorps contre une pluralité d'antigènes.

14. Procédé d'obtention d'une banque de lignées de cellules immortalisées qui produisent une pluralité d'anticorps monoclonaux d'intérêt, dont chacun se fixe à un antigène candidat différent, ledit procédé comprenant les étapes de :

   a) introduire une pluralité d'antigènes différents chez un animal non humain ou des animaux non humains ;
   b) récupérer des cellules produisant des anticorps à partir dudit animal ou desdits animaux et amener ces cellules dans une suspension à cellule unique ;
   c) générer des lignées de cellules immortalisées à partir de ladite suspension à cellule unique ;
   d) cribler le surnageant desdites lignées de cellules immortalisées contre une puce de protéine sur laquelle les antigènes candidats sont affichés ; et
   e) sélectionner des lignées de cellules immortalisées qui produisent des surnageants comprenant des anticorps qui se fixent auxdits antigènes candidats.

FIG. 1

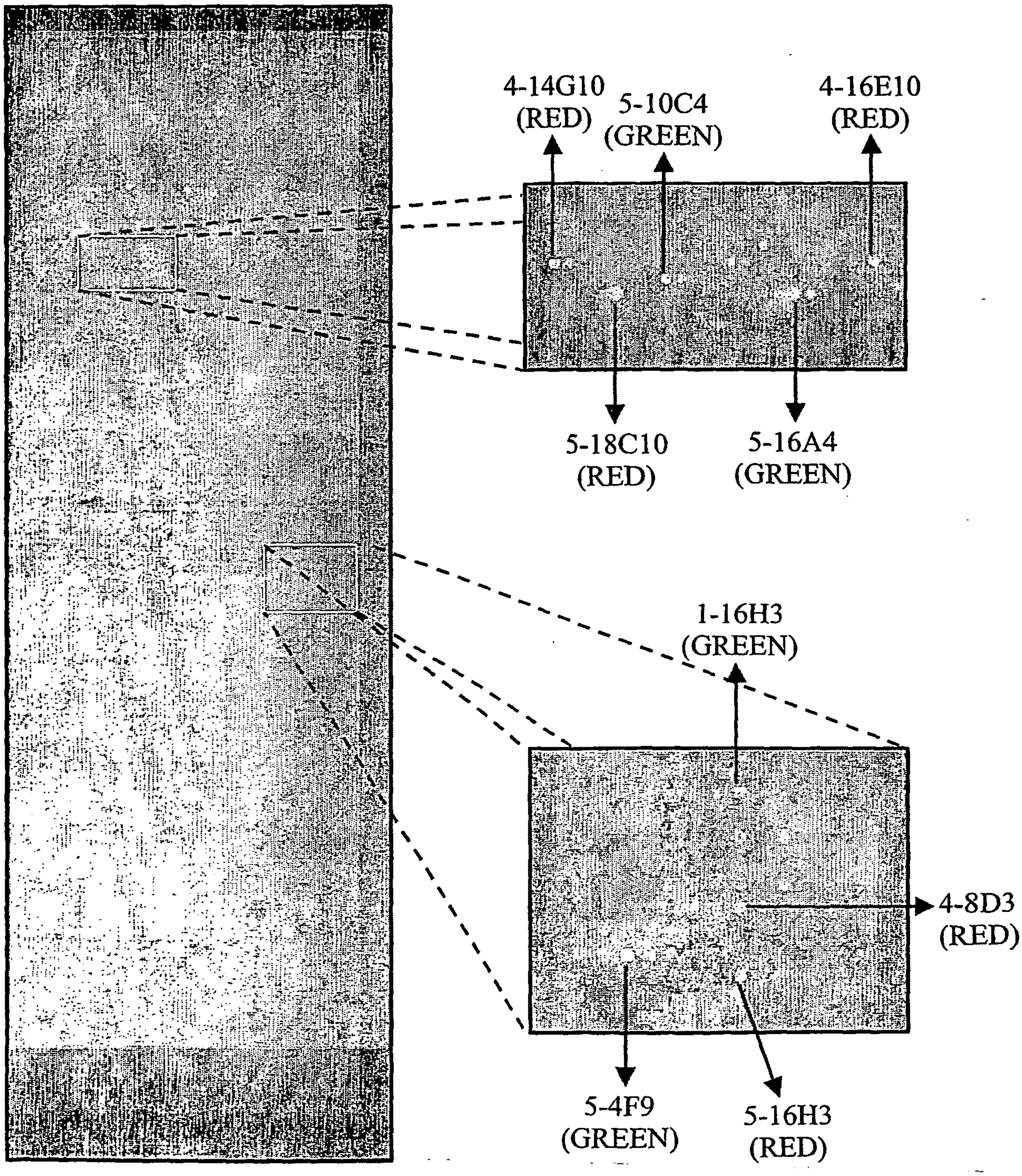
4-14G10
(RED)
5-10C4
(GREEN)
4-16E10
(RED)
5-18C10
(RED)
5-16A4
(GREEN)
1-16H3
(GREEN)
4-8D3
(RED)
5-4F9
(GREEN)
5-16H3
(RED)

FIG. 2

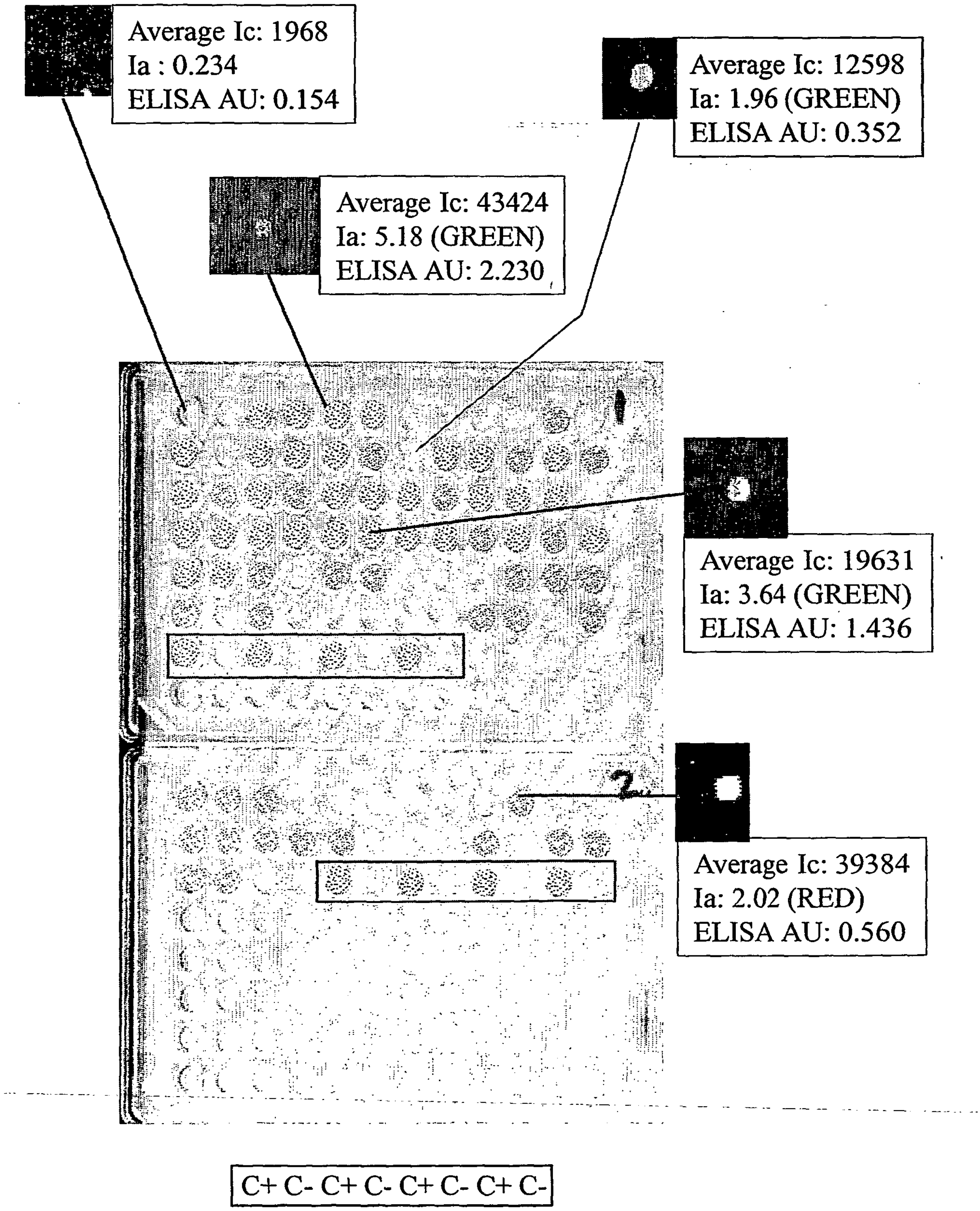
Average Ic: 1968
Ia : 0.234
ELISA AU: 0.154
Average Ic: 12598
Ia: 1.96 (GREEN)
ELISA AU: 0.352
Average Ic: 43424
Ia: 5.18 (GREEN)
ELISA AU: 2.230
Average Ic: 19631
Ia: 3.64 (GREEN)
ELISA AU: 1.436
Average Ic: 39384
Ia: 2.02 (RED)
ELISA AU: 0.560
C+ C- C+ C- C+ C- C+ C-

FIG. 3A

B5 Clone 1B6 (Normalised Data)

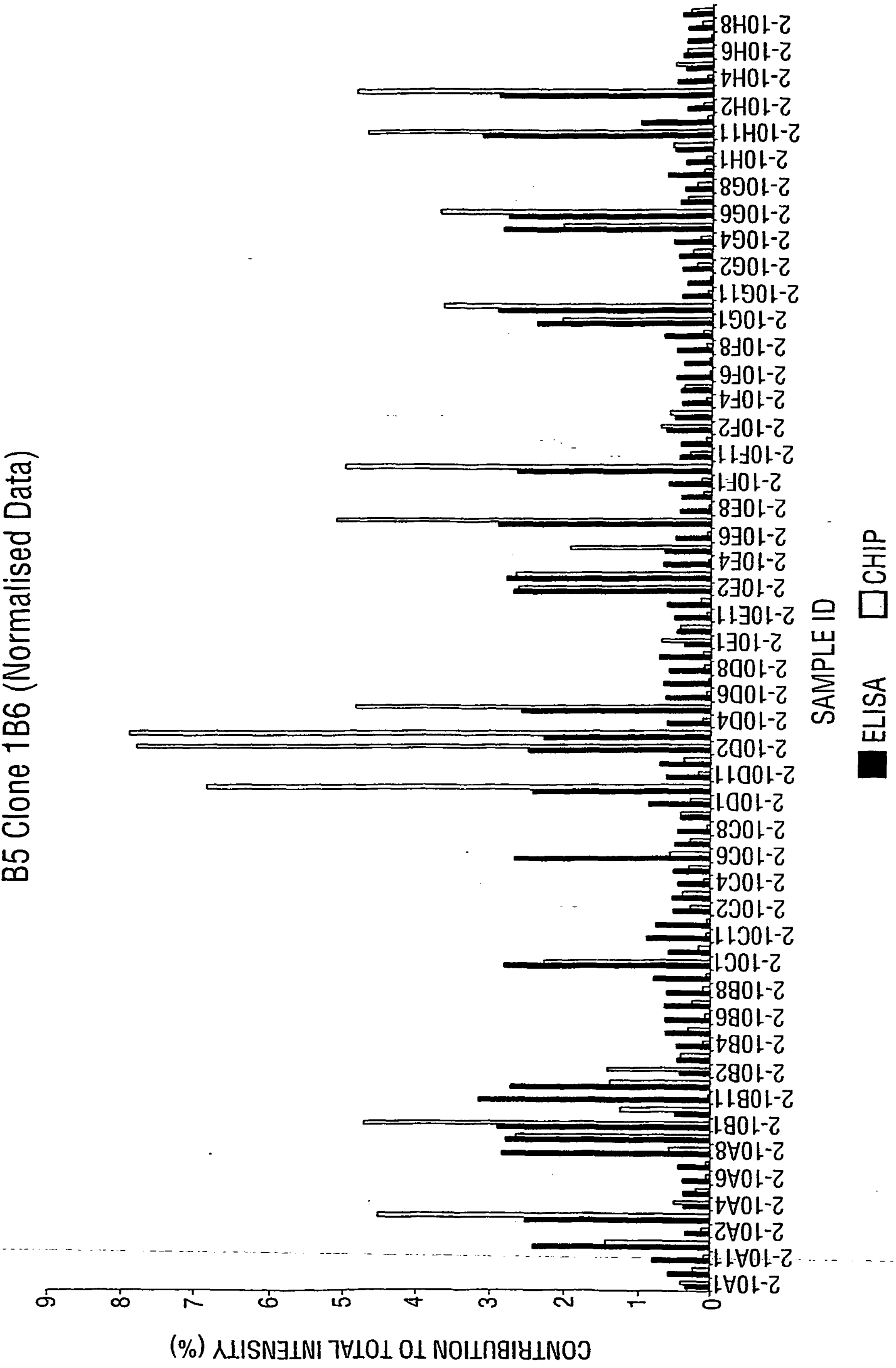

# FIG. 3B

B5 Clone 1B6 (Normalised Data)

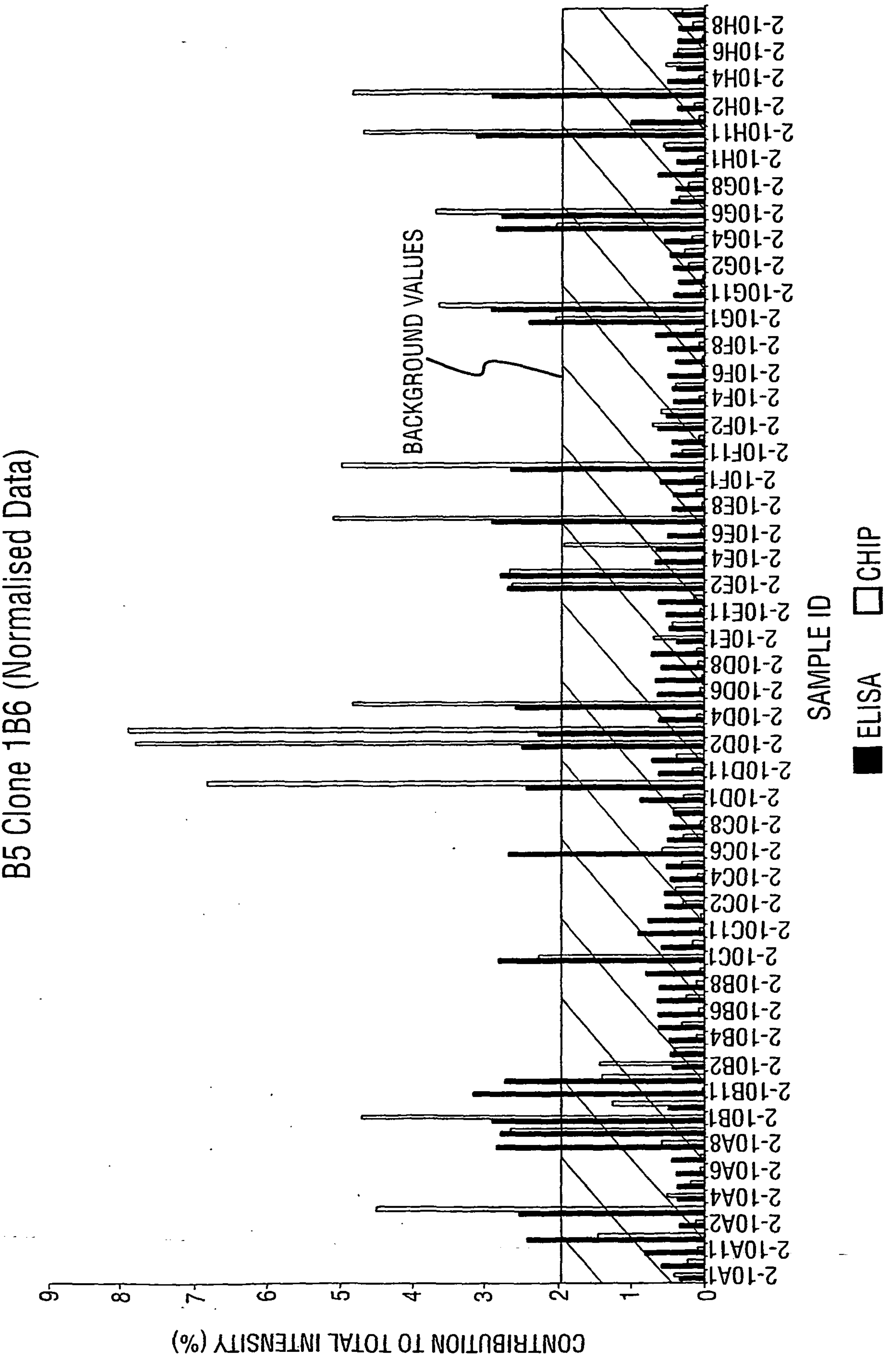

FIG. 4A

B5 Clone 5C5 (Normalised Data)

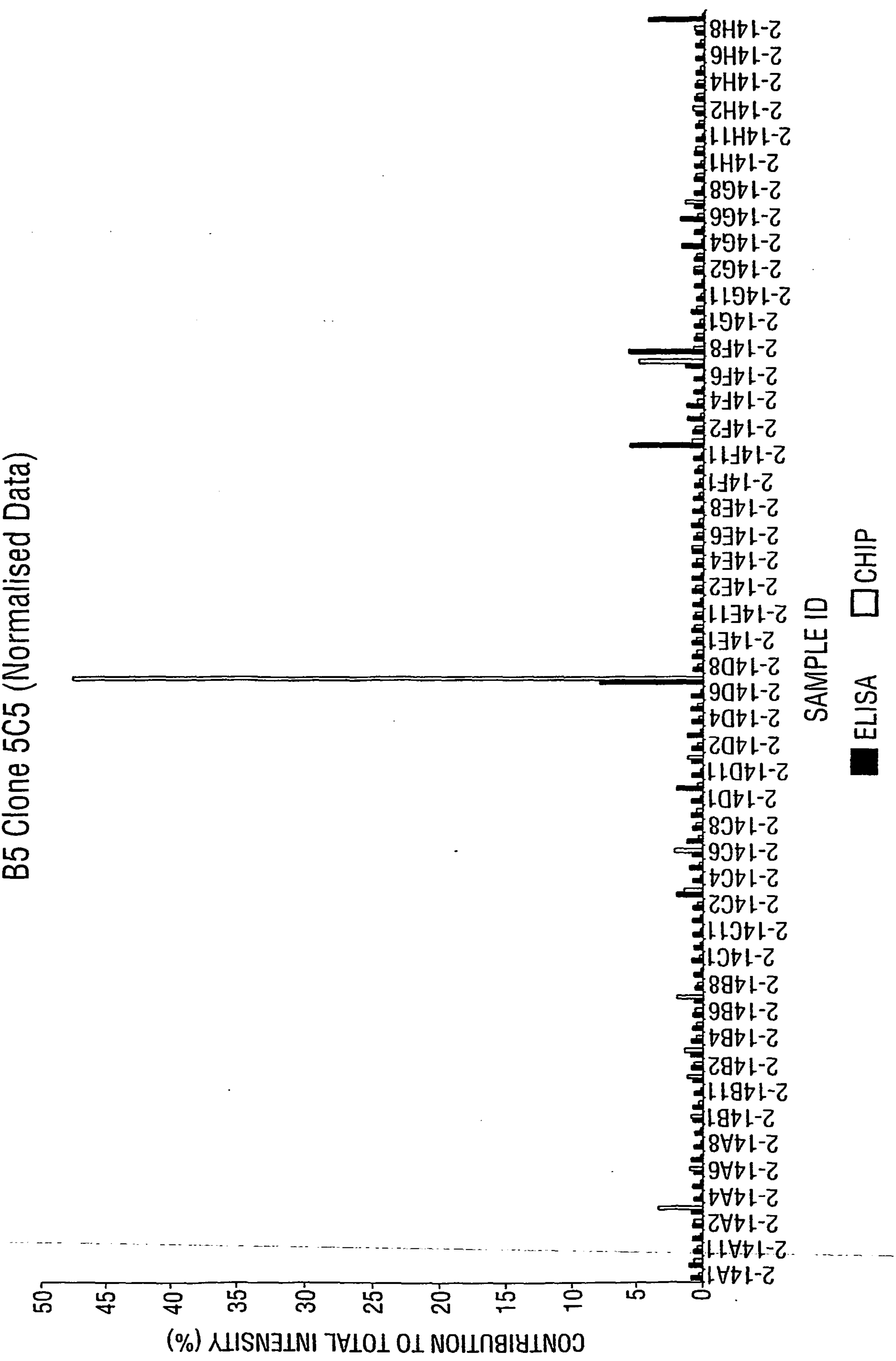

FIG. 4B

B5 Clone 5C5 (Normalised Data)

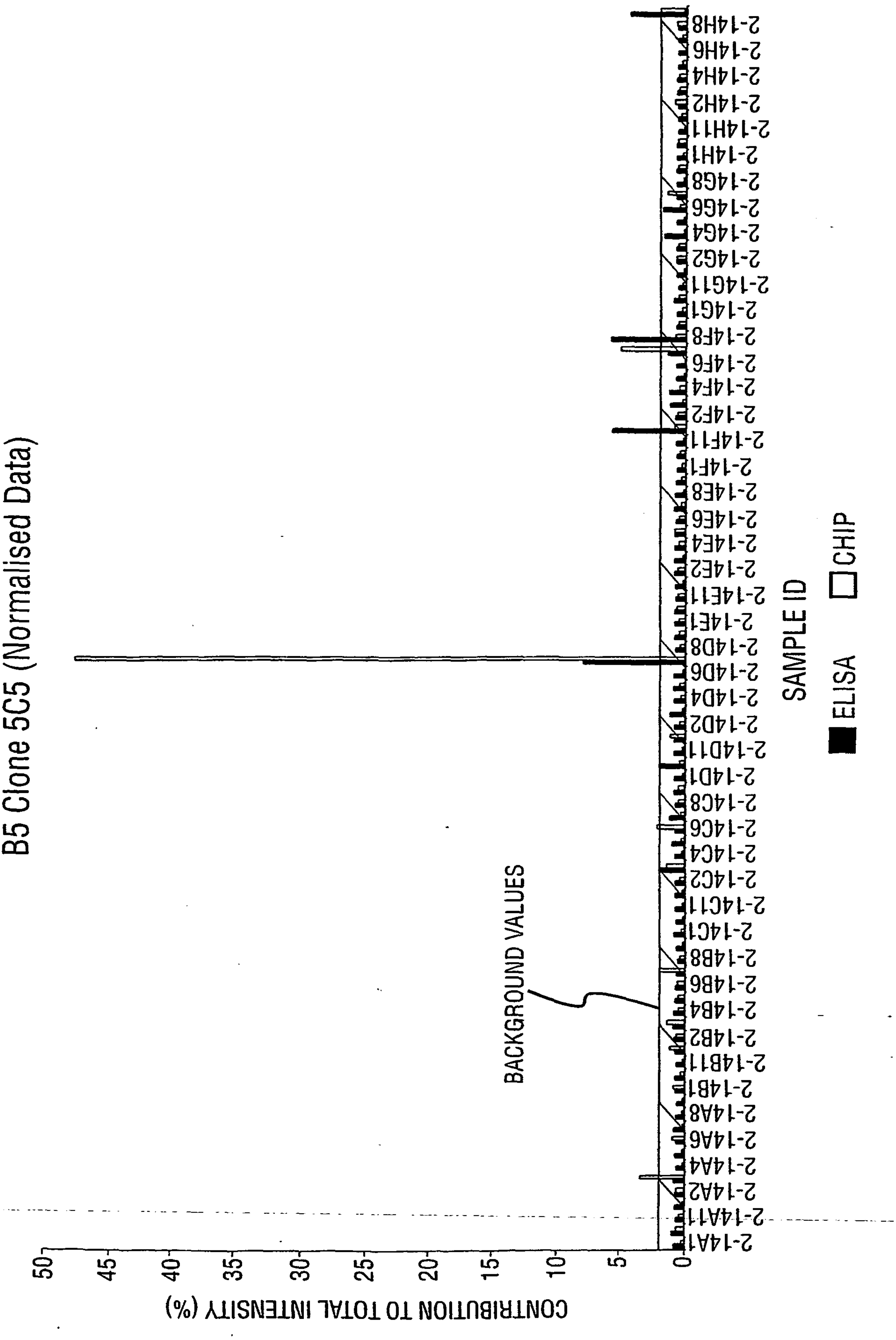

FIG. 5A

Ket94/95 Clone 8D1 (Normalised Data)

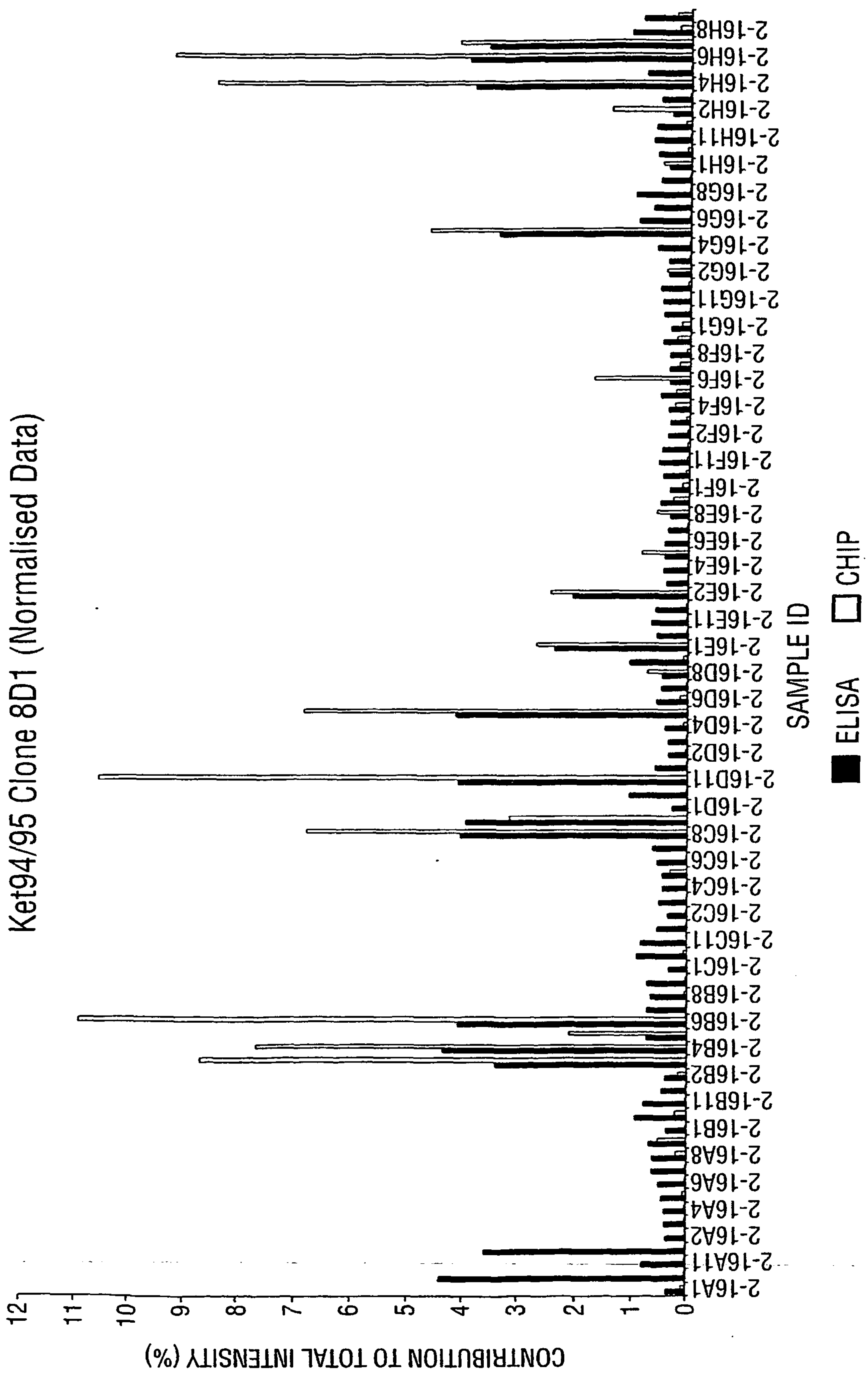

FIG. 5B

Ket94/95 Clone 8D1 (Normalised Data)

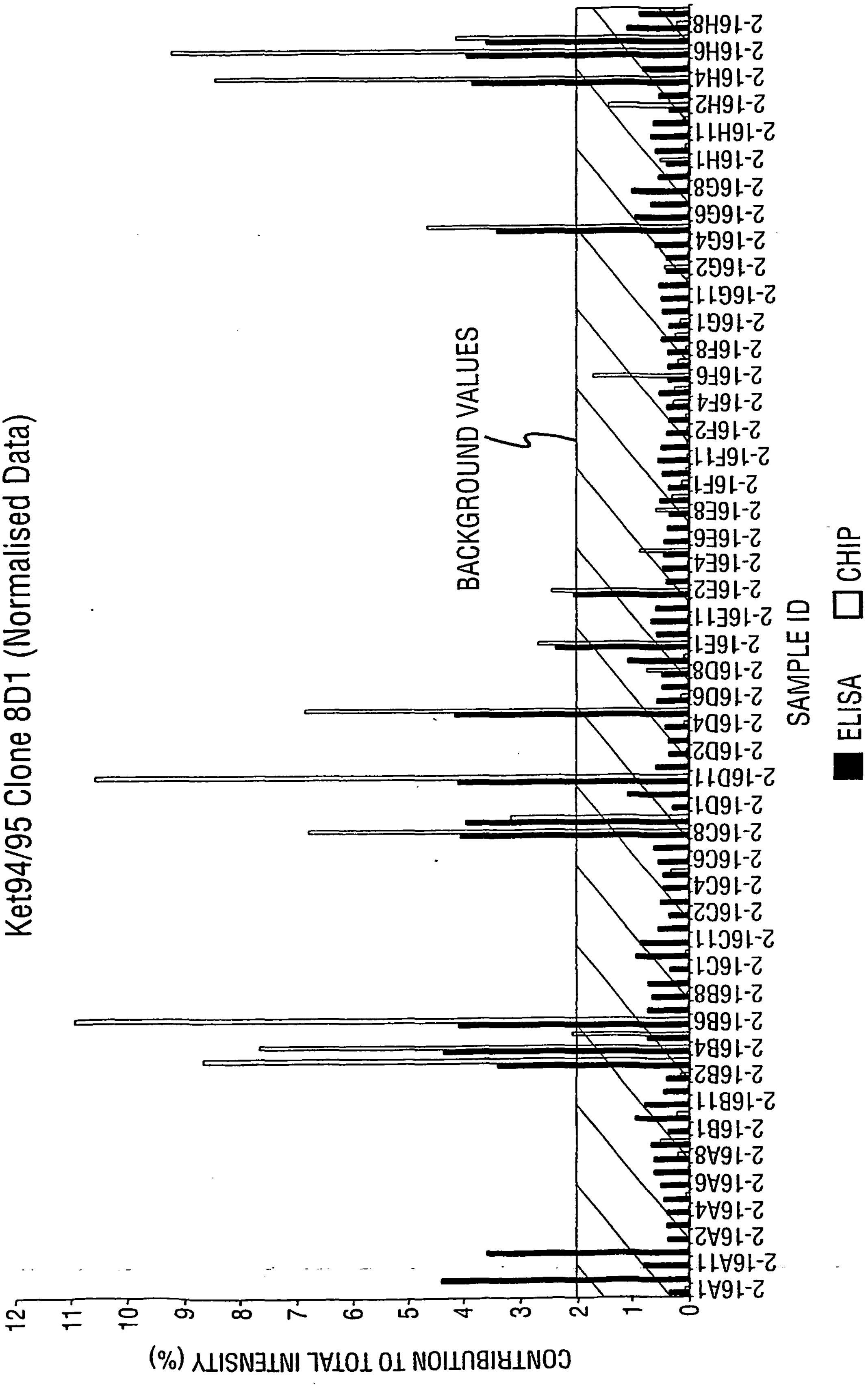

FIG. 6A

Ket94/95 Clone 8C6 (Normalised Data)

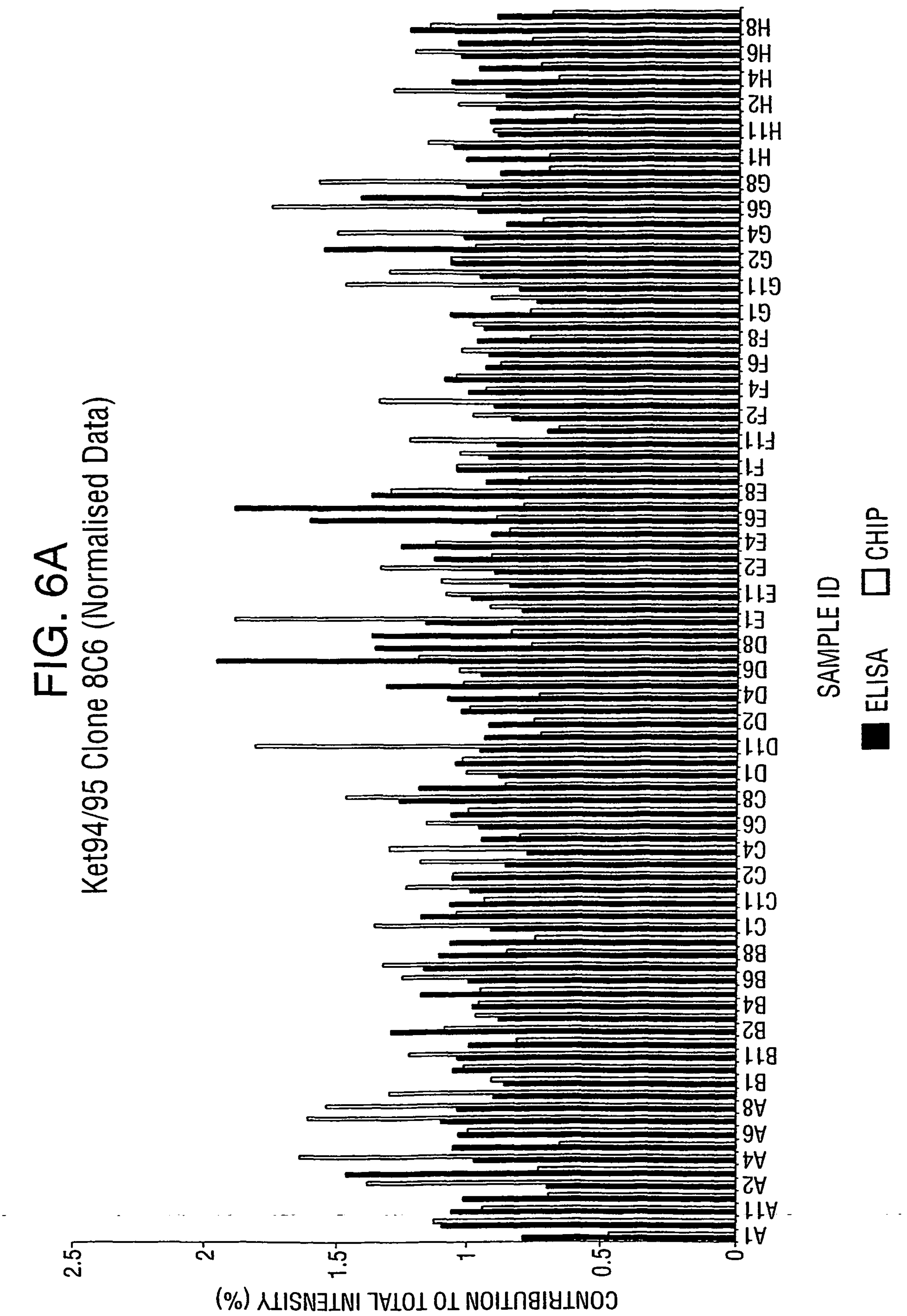

FIG. 6B

Ket94/95 Clone 8C6 (Normalised Data)